# EUROPEAN PATENT APPLICATION

(11) **EP 4 623 834 A1**
(43) Date of publication of application: **01.10.2025**
(21) Application number: 25165883.7
(22) Date of filing: 25.03.2025
(51) Int. Cl.: A61B 8/00, A61B 8/12

(54) **ULTRASOUND MODULAR FRONT-END FRAMEWORK**

(30) Priority: 27.03.2024 US 202418617678
(71) Applicant: Siemens Medical Solutions USA, Inc., Malvern, PA 19355 (US)
(72) Inventor: URBANO, Joseph A., Audubon, PA 19403 (US); SCHWARTZ KLESSEL, Jodi, North Wales, PA 19454 (US); DRESCHEL, William R., State College, PA 16803 (US)
(74) Representative: HKW Intellectual Property PartG mbB

(57) **Abstract**

An ultrasound modular front-end framework. An ultrasound modular front-end (114a, 114b) includes a first ultrasound front-end circuit (120a, 120b) that generates digital ultrasound data, and a communication module (122a, 122b) that is configured to be communicatively coupled to an ultrasound probe (130a, 130b) and a main console (101). The main console (101) constructs ultrasound images based on the digital ultrasound data. The ultrasound front-end circuit (120a, 120b) is disposed within a housing that is separate from a housing of the main console (101).

## Description

### TECHNICAL FIELD

The present disclosure generally relates to medical imaging, and more particularly to an ultrasound modular front-end framework.

### BACKGROUND

Ultrasound imaging systems generally operate according to a "pulse-echo method." Such systems must be capable of alternately transmitting and receiving of ultrasound signals. When transmitting, one or more piezoelectric transducer elements, for example, arranged in a linear or two-dimensional array, are excited to high-frequency oscillation by electrical pulses emitted by a transmitter, thereby generating an ultrasound pulse that may be directed at an object to be imaged. This ultrasound pulse is echoed back towards the transducer from some point within the object; for example, at boundary layers between two media with differing acoustic impedances. Then, when receiving, the "echo pulse" is received by the transducer element and converted into a corresponding electrical input signal (i.e., the "echo signal") that is fed to a receiver equipped with sensitive preamplifiers for enhancing the signal. The amplified signal may then be fed to a signal processor for evaluating the echoed image data to generate a visual image.

Ultrasound imaging systems may be divided into three components: (1) front-end (FE) that performs transmission, reception, and digitization of the ultrasound signal; (2) mid-end that performs demodulation, envelope detection, and compression; and (3) back-end that performs post-processing. The front-end of an ultrasound system is responsible for transmitting the ultrasound signal into the body and receiving the echoes that return from the body. The received echoes are then amplified and digitized for further processing.

Present-day ultrasound imaging systems can be classified into two categories: (1) systems with ultrasound front-end in the probe, or (2) systems with incorporated front-end. For convenience, the former will be referred to herein as hand-held systems, and the latter as cart-based systems. By this definition, cart-based systems also include portable systems, such as laptop style, with ports that connect to conventional ultrasound probes. The probes of hand-held systems may connect to mobile devices or other consoles that contain no front-end, or to consoles that also include a front-end of their own. For example, a console that operates both conventional cabled probes and wireless probes may have front-ends in the wireless probes as well as the console. Advanced applications and probe support are typically only provided by cart-based systems, while hand-held systems typically offer only reduced functionality because their front-end is limited by size and power constraints. Portable systems have been made to dock into carts, but the carts have not included ultrasound front-ends.

### SUMMARY

Described herein is an ultrasound modular front-end framework. An ultrasound modular front-end includes a first ultrasound front-end circuit that generates digital ultrasound data, and a communication module that is configured to be communicatively coupled to an ultrasound probe and a main console. The main console constructs ultrasound images based on the digital ultrasound data. The ultrasound front-end circuit is disposed within a housing that is separate from a housing of the main console.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.
FIG. 1 is a block diagram illustrating an exemplary ultrasound imaging system;
FIG. 2A illustrates an exemplary configuration of an ultrasound imaging system;
FIG. 2B illustrates an exemplary modular front-end (MFE);
FIG. 3 illustrates another exemplary configuration of an ultrasound imaging system; and
FIG. 4 shows an exemplary method of ultrasound imaging.

### DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order-dependent in their performance.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

In accordance with one aspect, the framework provides a versatile system architecture based on modular ultrasound front-end subsystems that enables an extensible ultrasound imaging platform that may function with varying levels of functionality and performance, depending on the intended clinical use. For example, the ultrasound imaging system may be configured as a portable system with only wireless probe support for basic guidance applications, but may be enhanced by the addition of a modular FE (MFE) subsystem to support more advanced cardiac applications. As another example, multiple system configurations may be produced from a common console or compute engine.

The advantages of the MFE-based system over prior art are efficiency, low cost, and scalability. A manufacturer may produce a common main console (MC) that provides the primary computing resources of the system. Multiple MFEs of varying cost, performance and/or probe support may also be produced by the manufacturer. The manufacturer may then create multiple system configurations tailored to certain clinical applications by combining the MC with one or more MFEs.

Alternatively, a basic product may be offered at a lower price point and enhanced by an added MFE. For example, the basic product may provide portability and basic point-of-care (POC) guidance capabilities. That same system may be "enhanced" to also provide advanced four-dimensional (4D) cardiac capability to guide structural heart procedures. The system vendor achieves economies of scale by selling the same base system for multiple clinical applications. The customer gets versatility by being able to use the same system for multiple applications, instead of having to purchase and support multiple, and often different, systems. These and other exemplary advantages and features will be described in more details in the following description.

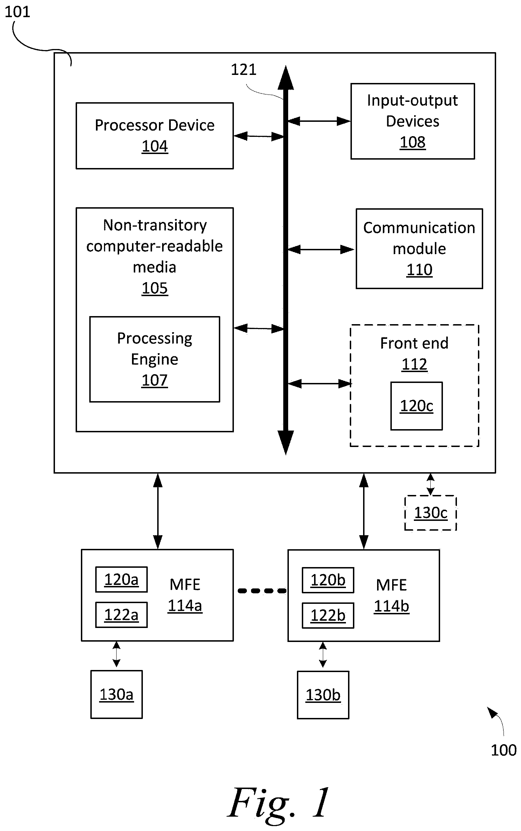

FIG. 1 is a block diagram illustrating an exemplary ultrasound imaging system 100. In some implementations, system 100 includes one or more modular front-ends (MFEs) (114a to 114b) communicatively coupled to a main console 101. Main console 101 may be enclosed in a housing that is separate from the housing of each MFE (114a or 114b). Main console 101 and/or modular front-ends (MFEs) (114a to 114b) may be portable and/or compact devices, requiring minimal size and power.

Main console 101 includes a processor device 104 coupled to one or more non-transitory computer-readable media 105 (e.g., computer storage or memory device), input-output devices 108 (e.g., monitor, mouse, touchpad or keyboard), communication module 110 and an optional native front-end 112 via an input-output interface 121. Processor device 104 may include, for example, a central processing unit (CPU), a graphical processing unit (GPU), field-programmable gate array (FPGA), or a combination thereof. Main console 101 may further include support circuits such as a cache, a power supply or battery, clock circuits, and a communications bus (not shown). Various other peripheral devices, such as additional data storage devices and printing devices, may also be connected to the main console 101. Additional computing resources (e.g., CPU, GPU, FPGA, computer storage) may be made available to the main console 101 for enhanced computational capacity. Such computing resources may reside within, or coupled to, an MFE (114a or 114b). These additional computing resources may be used to enhance the functionality of the main console 101, especially in support of the added functionality (e.g., support of a new probe type, like a matrix array) provided by the MFE (114a or 114b) itself.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing engine 107. Non-transitory computer-readable media 105 may include random access memory (RAM), read-only memory (ROM), magnetic floppy disk, flash memory, and other types of memories, or a combination thereof. The computer-readable program code is executed by processor device 104 to process data acquired by, for example, MFEs 114a-114b. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database, including, but not limited to, image datasets, a knowledge base, individual subject data, medical records, diagnostic reports (or documents) for subjects, or a combination thereof.

Communication module 110 enables the main console 101 to communicate with MFEs 114a-114b and/or other external systems and/or networks. In some implementations, communication module 110 includes a high-speed digital interface, such as Thunderbolt^{™}, universal serial bus (USB), multi-Gigabit Ethernet, optical fiber, waveguide technology, or a wireless interface. Other types of interfaces are also useful. In some implementations, communication module 110 includes wireless signal transceiver that communicates signals using a common communication protocol, such as Global System for Mobile Communications (GSM), WIFI, Bluetooth, Zigbee, LoRa, and TCP/IP.

MFEs 114a-114b are configured to acquire digital ultrasound data. Each MFE (114a, 114b) includes an ultrasound front-end (FE) circuit (120a or 120b) that performs transmission, reception, and digitization of ultrasound signals, and communication module (122a, 122b) for communicating the ultrasound data. Other components, such as additional computing resources, may also be included in MFEs (114a-114b). In some implementations, the front-end circuit (120a,120b) includes a transmitter, a receiver, and an analog-to-digital converter (ADC). The transmitter excites or triggers a transducer with transmit pulses to transmit ultrasound waves into a subject region. The transducer receives ultrasound waves from the subject region in response to the ultrasound waves transmitted into the subject region. The receiver receives analog echo signals from the transducer in response to the ultrasound waves and amplifies them. The ADC converts the analog echo signals to digital ultrasound data. In some implementations, FE circuit (120a, 120b) facilitates partial or full beamforming on either transmit or receive. In the case of partial beamforming, further beamforming may take place outside of the MFE (114a, 114b), for example, within the main console 101.

Each MFE (114a, 114b) is self-contained in its own housing external to the housing of the main console 101. The housing of each MFE (114a, 114b) is plastic, metal, wood, fiberglass, or any other now-known or later-developed material for housing electronics. The respective FE circuit (120a, 120b) is disposed within the MFE housing. MFE housing may further at least partially house a communication module (122a, 122b), such as covering a portion of the communication module (122a, 122b) and allowing access to one or more ports for electrical connection.

Communication module (122a, 122b) includes first and second communication interfaces configured to be communicatively coupled to ultrasound probe (130a, 130b) and main console 101. First and second communication interfaces may include a wired interface such as a high-speed digital wired interface (e.g., Thunderbolt^{™}, USB, multi-Gigabit Ethernet, optical fiber, waveguide technology). For example, a given probe (130a, 130b) may be connected to one of the first communication interfaces by attaching its connector to a port of the first communication interface. An interface cable or other suitable electrical connection may be used to connect the first communication interface to the probe (130a, 130b). For example, a micro-coaxial cable may be used to carry analog signals between a front-end transmit/receive channel to a transducer in the probe (130a, 130b). Other connection schemes are also possible, including digital data interfaces.

A cable connection or docking connection may be used to connect the second communication interface to the main console 101. Alternatively, first and second communication interfaces may include a wireless interface. In some implementations, communication module (122a, 122b) includes a wireless receiver for bridging communication between a wireless probe 130 and main console 101. In the case of a wired communication interface connectable to a cable, the MFE (114a, 114b) may be powered by an external power supply over the same cable using technology such as Power-Over-Ethernet (POE) or the like. Alternatively, the MFE housing may further provide a power input port that is connected to the external power supply.

Probe (130a, 130b) is communicatively coupled to the MFE (114a, 114b) via a wired or wireless connection. Probe (130a, 130b) includes an ultrasound transducer that transmits and receives ultrasound waves from the subject region. The ultrasound transducer may include an array of piezoelectric, capacitive membrane ultrasound transducer or other now-known or later-developed elements for converting between electrical and acoustical energies. In some implementations, the transducer is housed in a probe housing. The probe housing may be shaped for hand-held use. In other implementations, the probe housing is shaped for use internal to a patient, such as shaped as an endoscope or catheter. In some implementations, the probe housing at least partially houses an array of transducers, such as covering a portion of the transducer array and allowing a face of the array acoustical access for scanning a subject. In some implementations, probe (130a, 130b) includes its own ultrasound FE circuit, which may perform receive and/or transmit beamforming.

A native ultrasound front-end (FE) 112 may optionally be provided in the main console 101. Ultrasound front-end 112 includes an ultrasound front-end (FE) circuit 120c that performs transmission, reception, and digitization of the ultrasound signal from the probe 130c to output digital ultrasound data. Probe 130c is communicatively coupled to the front-end 112 via a wired or wireless connection. In some implementations, ultrasound front-end 112 may have limited or different capability from the front-end circuits 120a-120b of the MFEs 114a-114b. For example, ultrasound FE circuit 120c may support a probe 130c that is a different type from probes 130a-130b that are supported by MFEs 114a-114b. In this case, the main console 101 may form an imaging system in some limited capacity, using its native FE 112 with probe 130c. The additional MFEs 114a-114b may provide support for additional probes 130a-130b not otherwise supported by the front-end 112.

It should be appreciated that the main console 101 does not need a FE circuit 120c of its own. Main console 101 may operate only through the connection of one or more MFEs 114a-114b. Alternatively, main console 101 may operate with wireless and/or digital probes that contain their own FE. In the former case, the MFE (114a, 114b) is an essential component of the ultrasound system formed by the combination of the probe (130a, 130b), the MFE (114a, 114b), and the main console 101. In the latter case, the MFE (114a, 114b) is an optional enhancement to the system formed by the main console 101 and its wireless and/or digital probes. In this case, the system may function as an imaging system without the MFE (114a, 114b), but at some reduced capacity. For example, the added MFE (114a, 114b) may provide support for additional probes (130a, 130b) not otherwise supported by the main console 101.

In some implementations, MFEs 114a-114b form the basis of a distributed system, wherein the MFEs 114a-114b and main console 101 are physically separated by a distance (e.g., at least 3 feet). In this case, a common main console 101 may serve multiple MFEs 114-114b located in different rooms or different areas of a building. The MFE(s) 114-114b may be located in a procedure area for scanning patients. Main console 101 may be located relatively remotely from the procedure areas. This may be useful in environments that are inhospitable to the presence of a main console 101.

In some implementations, main console 101 is configured to process digital ultrasound data streams from multiple MFEs 114a-114b simultaneously, producing independent images, video and/or audio from each MFE's ultrasound data stream. The image, video and/or audio data may be sent back to the procedure areas through either a cabled or wireless communication interface to be presented. Locating the main console 101 in a remote location while centralizing the processing of ultrasound data streams from multiple MFEs 114a-114b may provide benefits in processing power by allowing main console 101 to be physically large and power consumptive relative to the MFEs 114a-114b. This may enable the expanded use of artificial intelligence or other advanced diagnostic tools. A further enhancement may be to include a wireless probe receiver in the MFE 114a-114b. Accordingly, the MFE 114a-114b also acts as a bridge between a wireless probe and the main console 101, which is a useful feature given that the main console 101 may be located relatively remotely from the procedure area.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

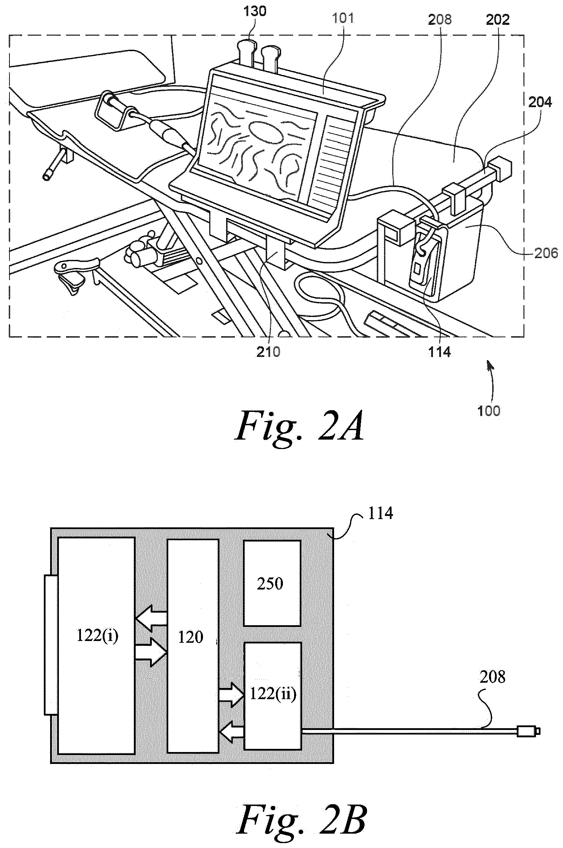

FIG. 2A illustrates an exemplary configuration of an ultrasound imaging system 100. As illustrated, MFE 114 is detachably mounted to a rail 204 of a patient's procedure table (e.g., bed) 202 in an interventional suite. It should be appreciated that MFE 114 may also be detachably mounted in other positions near the patient's procedure table 202, such as on a wall, an intravenous pole, a shelf, a table or the like. A bracket 206 provides the mechanical support for detachably mounting a housing of the MFE 114 to a rail 204. MFE 114 is communicatively coupled to main console 101 via cable 208. Main console 101 is detachably mounted on the patient's procedure table 202 using brackets 210. Main console 101 may also be mounted in other positions (e.g., a wall, an intravenous pole, a shelf, a table) near the patient's procedure table 202. Such configurations are advantageously "zero footprint", because they keep equipment from cluttering the procedure area.

One or more types of ultrasound probes 130 may be communicatively coupled to the MFE 114. The configuration illustrated in FIG. 2A is especially useful for interventional cardiology, particularly for electrophysiology (EP) and structural heart applications. For the former, an intracardiac echocardiography (ICE) catheter may be plugged into the MFE 114 at bedside, providing easy integration in the EP environment. The structural heart application may require either a matrix transesophageal echocardiography (TEE) or matrix ICE (MICE) probe. MFE 114 may support either of these types of probes. In some implementations, multiple probe connectors may be provided on the MFE 114 (e.g., on either side), for such applications that require the availability of multiple cabled probes. Alternatively, multiple MFEs 114 of different types suited to accommodate particular probes may be connected to the main console 101 at the same time. Such "connections" may be wireless or wired.

Both the matrix TEE and MICE examples demonstrate the advantages of this framework. Matrix TEE probes are very expensive and can operate on different ultrasound platforms. Users may want to reuse the same TEE probe in different environments, if possible. Moreover, they may prefer to use a portable or zero-footprint system in the interventional suite. MICE catheters are highly specialized, sterilizable transducers. They are typically designed to plug into an adapter cable for connection to a cart-based ultrasound system. MFE 114 may serve as an alternative "adapter" that allows the MICE catheters to connect to a main console 101 with no front-end or with an inadequate front-end of its own. In both the matrix TEE and MICE cases, some FE components may also be included in the probes themselves. These typically include transmitters and subarray receive beamformers. For these cases, MFE 114 may provide analog receivers, ADCs, and some support for transmit and receive beamforming. As mentioned previously, MFE 114 need not provide all typical FE features. At minimum, MFE 114 may include analog transmitters, receivers, and ADCs, as well as communication interfaces to the main console 101 and probe 130.

FIG. 2B illustrates an exemplary MFE 114. MFE 114 may include an ultrasound front-end (FE) circuit 120, first and second communication interfaces 122(i) and 122(ii), and power supply 250 for providing power. First communication interface 122(i) may include, for example, a multi-channel transducer port connector for communicating with an ultrasound probe 130, such as a TEE probe or MICE catheter. First communication interface 122(i) communicates ultrasound signals received from the ultrasound probe 130 to ultrasound FE circuit 120.

In some implementations, ultrasound FE circuit 120 includes a transmitter (e.g., multi-channel pulser), receiver and ADC. For example, a multi-channel pulser may be used to transmit pulses to excite the probe 130, which transmits ultrasound waves into a subject region. The receiver that receives analog echo signals from the probe 130 in response to the ultrasound waves and amplifies them. The ADC converts the analog echo signals to digital ultrasound data. The FE circuit 120 may then communicate the ultrasound data to second communication interface 122(ii) that sends the ultrasound data to the main console 101. Second communication interface 122(ii) may include, for example, a control and data sequencer that sends the ultrasound data via a high-speed data link 208.

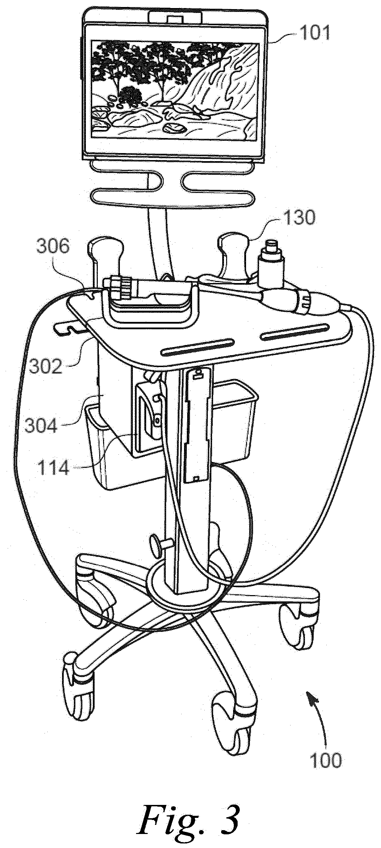

FIG. 3 illustrates another exemplary configuration of an ultrasound imaging system 100. MFE 114 is detachably mounted to a wheel assembly 302. MFE 114 is mounted in a support bracket 304 under a surface 306 of the wheel assembly 302. Main console 101 is a portable display unit that is positioned on the surface 306 and communicatively coupled to MFE 114. MFE 114 is communicatively coupled to probes 130, which may be stored on surface 130. It should be appreciated that other system configurations are also possible. For example, the main console 101 may be compact and configured to be hand-held. MFE 114 may reside on a cart or some fixed location. Main console 101 may be docked onto a cart or other fixed location, thereby extending its capability.

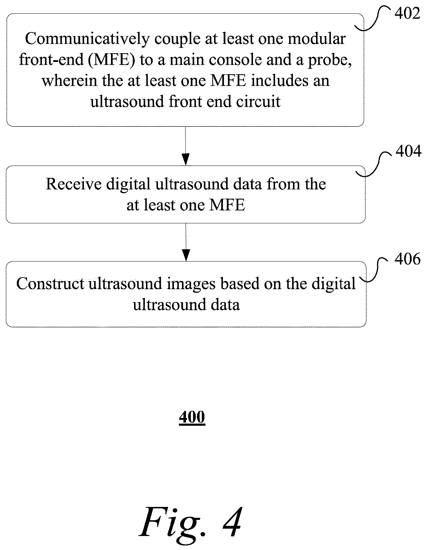

FIG. 4 shows an exemplary method 400 of ultrasound imaging. It should be understood that the steps of the method 400 may be performed in the order shown or a different order. Additional, different, or fewer steps may also be provided. Further, the method 400 may be implemented with system 100 of FIGS. 1-3, a different system, or a combination thereof.

At 402, at least one modular front-end (MFE) (114a, 114b) is communicatively coupled to a main console 101 and a probe (130a, 130b). MFE (114a, 114b) is enclosed in its own MFE housing that is separate from the housing of the main console 101. An FE circuit (120a, 120b) is disposed in the MFE housing. The FE circuit (120a, 120b) may include a transmitter, a receiver and an analog-to digital converter (ADC) that generates ultrasound data in response to ultrasound signals from the probe (130a, 130b). MFE housing may further provide a communication module (122a, 122b) including a first communication interface communicatively coupled to an ultrasound probe (130a, 130b) and second communication interface communicatively coupled to the main console 101. The first and second communication interfaces may include a wired (or cabled) or wireless interface.

Probe (130a, 130b) may include an array of transducers that convert acoustic energies to electrical energies. The one or more MFEs (114a-114b) may provide support for additional probes not otherwise supported by main console 101.

At 404, processing engine 107 in the main console 101 receives digital ultrasound data from the MFE (114a, 114b). Processing engine 107 in main console 101 may be versatile, and support various types of probes (130a-c), imaging formats and/or image processing methods. For example, the probes (130a-c) may be wireless or wired, have their own front-ends or not, matrix TEE, ICE or other specialized ultrasound probes. Processing engine 107 in main console 101 may provide enhanced transducer support when attached to the respective MFE (114a, 114b), but disable such support while the main console 101 is detached from the MFE (114a, 114b).

At 404, processing engine 107 in the main console 101 constructs ultrasound images based on the digital ultrasound data. The digital ultrasound may be processed further before displayable ultrasound images are created. Some image construction tasks are also referred to as "beam formation tasks". Processing engine 107 may perform full, partial or no beamforming based on the received digital ultrasound data. Further processing tasks, such as sampling, detection and/or formatting, may be performed by the processing engine 107 to generate displayable ultrasound images. Such ultrasound images may be displayed on, for example, a monitor 108 communicatively coupled to the main console 101 and/or another monitor communicatively coupled to the MFE (114a, 114b).

The following is a list of non-limiting illustrative embodiments disclosed herein:
Illustrative embodiment 1. An ultrasound modular front-end, comprising:
a first ultrasound front-end circuit that generates digital ultrasound data; a communication module that is configured to be communicatively coupled to a first ultrasound probe and a main console, wherein the main console constructs ultrasound images based on the digital ultrasound data; and a first housing separate from a second housing of the main console, wherein the first ultrasound front-end circuit is disposed within the first housing.

Illustrative embodiment 2. The ultrasound modular front-end of illustrative embodiment 1, wherein the first ultrasound front-end circuit comprises a transmitter, a receiver and an analog-to-digital converter.

Illustrative embodiment 3. The ultrasound modular front-end of any one of illustrative embodiments 1-2 wherein the first ultrasound front-end circuit facilitates partial or full beamforming.

Illustrative embodiment 4. The ultrasound modular front-end of any one of illustrative embodiments 1-3 wherein the communication module comprises first and second communication interfaces configured to be communicatively coupled to the first ultrasound probe and the main console respectively.

Illustrative embodiment 5. The ultrasound modular front-end of any one of illustrative embodiments 1-4 wherein the first ultrasound probe includes a second ultrasound front-end circuit.

Illustrative embodiment 6. The ultrasound modular front-end of any one of illustrative embodiments 1-5 wherein the main console includes a third ultrasound front-end circuit that supports a second ultrasound probe that is a different type from the first ultrasound probe.

Illustrative embodiment 7. The ultrasound modular front-end of any one of illustrative embodiments 1-6 further comprises one or more additional computing resources that are made available to the main console for enhanced computational capacity.

Illustrative embodiment 8. The ultrasound modular front-end of any one of illustrative embodiments 1-7 wherein the first housing is detachably mounted to a procedure table.

Illustrative embodiment 9. The ultrasound modular front-end of any one of illustrative embodiments 1-8 wherein the first ultrasound probe comprises an intracardiac echocardiography (ICE) catheter.

Illustrative embodiment 10. The ultrasound modular front-end of any one of illustrative embodiments 1-9 wherein the first ultrasound probe comprises a matrix transesophageal echocardiography (TEE) or matrix ICE (MICE) probe.

Illustrative embodiment 11. The ultrasound modular front-end of any one of illustrative embodiments 1-10 wherein the first housing is detachably mounted to a wheel assembly.

Illustrative embodiment 12. An ultrasound imaging system, comprising:
at least one modular front-end, including a communication module and a first ultrasound front-end circuit that generates digital ultrasound data; and a main console communicatively coupled to the at least one modular front-end via the communication module, wherein the main console constructs ultrasound images based on the digital ultrasound data, wherein the main console and the at least one modular front-end are physically separated.

Illustrative embodiment 13. The ultrasound imaging system of illustrative embodiment 12 wherein the at least one modular front-end comprises multiple modular front-ends.

Illustrative embodiment 14. The ultrasound imaging system of illustrative embodiment 13 wherein the main console processes the digital ultrasound data from the multiple modular front-ends simultaneously.

Illustrative embodiment 15. The ultrasound imaging system of any one of illustrative embodiments 13-14 wherein the multiple modular front-ends support different types of probes.

Illustrative embodiment 16. The ultrasound imaging system of any one of illustrative embodiments 12-15 wherein the communication module comprises a wireless receiver that enables an ultrasound probe to communicate wirelessly with the at least one modular front-end.

Illustrative embodiment 17. The ultrasound imaging system of any one of illustrative embodiments 12-16 wherein the at least one modular front-end and the main console are detachably mounted to a procedure table.

Illustrative embodiment 18. The ultrasound imaging system of any one of illustrative embodiments 12-17 wherein the at least one modular front-end is detachably mounted to a wheel assembly.

Illustrative embodiment 19. The ultrasound imaging system of illustrative embodiment 18 wherein the main console is a portable display unit that is positioned on a surface of the wheel assembly.

Illustrative embodiment 20. A method of ultrasound imaging, comprising: communicatively coupling at least one modular front-end (MFE) with a main console and a probe, wherein the at least one MFE includes an ultrasound front end circuit that generates digital ultrasound data in response to ultrasound signals from the probe; receiving, by the main console, the digital ultrasound data from the at least one MFE; and
constructing, by the main console, ultrasound images based on the digital ultrasound data.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

## Claims

1. An ultrasound modular front-end (114a, 114b), comprising:
a first ultrasound front-end circuit (120a, 120b) that generates digital ultrasound data;
a communication module (122a, 122b) that is configured to be communicatively coupled to a first ultrasound probe (130a, 130b) and a main console (101), wherein the main console (101) constructs ultrasound images based on the digital ultrasound data; and
a first housing separate from a second housing of the main console (101), wherein the first ultrasound front-end circuit (120a, 120b) is disposed within the first housing.

2. The ultrasound modular front-end (114a, 114b) of claim 1, wherein the first ultrasound front-end circuit (120a, 120b) comprises a transmitter, a receiver and an analog-to-digital converter.

3. The ultrasound modular front-end (114a, 114b) of claim 1 or 2, wherein the first ultrasound front-end circuit (120a, 120b) facilitates partial or full beamforming.

4. The ultrasound modular front-end (114a, 114b) of one of claims 1 to 3, wherein the communication module (122a, 122b) comprises first and second communication interfaces configured to be communicatively coupled to the first ultrasound probe (130a, 130b) and the main console (101) respectively.

5. The ultrasound modular front-end (114a, 114b) of one of claims 1 to 4, wherein the first ultrasound probe (130a, 130b) includes a second ultrasound front-end circuit.

6. The ultrasound modular front-end (114a, 114b) of one of claims 1 to 5, further comprises one or more additional computing resources that are made available to the main console (101) for enhanced computational capacity.

7. The ultrasound modular front-end (114a, 114b) of one of claims 1 to 6, wherein the first ultrasound probe (130a, 130b) comprises an intracardiac echocardiography (ICE) catheter.

8. The ultrasound modular front-end (114a, 114b) of one of claims 1 to 7, wherein the first ultrasound probe (130a, 130b) comprises a matrix transesophageal echocardiography (TEE) or matrix ICE (MICE) probe.

9. An ultrasound imaging system (100), comprising:
at least one modular front-end (114a, 114b), including a communication module (122a, 122b) and a first ultrasound front-end circuit (120a, 120b) that generates digital ultrasound data; and
a main console (101) communicatively coupled to the at least one modular front-end (114a, 114b) via the communication module (122a, 122b), wherein the main console (101) constructs ultrasound images based on the digital ultrasound data, wherein the main console (101) and the at least one modular front-end (114a, 114b) are physically separated.

10. The ultrasound imaging system (100) of claim 9, wherein the at least one modular front-end (114a, 114b) comprises multiple modular front-ends.

11. The ultrasound imaging system (100) of claim 10, wherein the main console (101) processes the digital ultrasound data from the multiple modular front-ends simultaneously.

12. The ultrasound imaging system (100) of one of claims 9 to 11, wherein the communication module (122a, 122b) comprises a wireless receiver that enables an ultrasound probe (130a, 130b) to communicate wirelessly with the at least one modular front-end (114a, 114b).

13. The ultrasound imaging system (100) of one of claims 9 to 12, wherein the at least one modular front-end (114a, 114b) and the main console (101) are detachably mounted to a procedure table.

14. The ultrasound imaging system (100) of one of claims 9 to 13, wherein the at least one modular front-end (114a, 114b) is detachably mounted to a wheel assembly.

15. The ultrasound imaging system (100) of claim 14, wherein the main console (101) is a portable display unit that is positioned on a surface of the wheel assembly.
